# EUROPÄISCHE PATENTANMELDUNG

(11) **EP 4 123 033 A1**
(43) Veröffentlichungstag der Anmeldung: **25.01.2023**
(21) Anmeldenummer: 21187066.2
(22) Anmeldetag: 21.07.2021
(51) Int. Cl.: C12Q 1/6806, C12Q 1/686

(54) **BLUTMATRIX ALS REFERENZMATERIAL FÜR DIE IN-VITRO DIAGNOSTIK**

(71) Anmelder: SensID GmbH, 18057 Rostock (DE)
(72) Erfinder: NOWACK, Björn, 18119 Seebad-Warnemünde (DE); LÜKING, Angelika, 14974 Ludwigsfelde (DE)
(74) Vertreter: Simandi, Claus

(57) **Zusammenfassung**

Die vorliegende Erfindung betrifft sowohl ein neues Referenzmaterial auf Basis einer Blutmatrix zur Verwendung in der Diagnostik, bzw. in-vitro Diagnostik, insbesondere solche mithilfe von Sequenzierverfahren von Nukleinsäuren, wie Polymerase-Chain-Reaction (PCR) oder Next Generation Sequencing (NGS) als auch ein Verfahren zu dessen Herstellung samt Validierung, insbesondere einer nicht-invasiven Liquid Biopsy (Flüssigkeitsbiopsien).

## Beschreibung

Die vorliegende Erfindung betrifft sowohl ein neues Referenzmaterial auf Basis einer Blutmatrix zur Verwendung in der Diagnostik, bzw. in-vitro Diagnostik, insbesondere solche mithilfe von Sequenzierverfahren von Nukleinsäuren, wie Polymerase-Chain-Reaction (PCR) oder Next Generation Sequencing (NGS) als auch ein Verfahren zu dessen Herstellung samt Validierung, insbesondere einer nicht-invasiven Liquid Biopsy (Flüssigkeitsbiopsien).

Gegenwärtige Verfahren zum Nachweis von Tumoren wie klinischchemische Untersuchungen in Körperflüssigkeiten und Gewebeproben oder bildgebende Verfahren führen trotz aller Fortschritte in den letzten Jahren zu einem meist zu späten Nachweis von malignen Veränderungen. So ist die Hauptursache der hohen Mortalitätsrate von Tumorpatienten nicht das Auftreten von Primärtumoren, sondern von Metastasen. Um das Auftreten von Metastasen zu verhindern, ist ein so früh wie möglicher Nachweis von malignen Veränderungen erforderlich. Ferner bedarf es Verfahren, um maligne Zellen von normalen Zellen richtig unterscheiden zu können. Sowohl falsch-positive als auch falsch-negative Befunde haben fatale Konsequenzen für die Betroffenen. Ebenso sind Verfahren erforderlich, die eine richtige Prognose von Tumorpatienten gestatten und eine entsprechend auf den Patienten individuell abgestimmte Therapie erlauben.

Bisher werden in Körperflüssigkeiten wie Blut-, Urin-, Sputum- oder Gewebeproben sogenannte Tumormarker, insbesondere Onkogene bestimmt. Hierbei handelt es sich um Bestandteile von Tumorzellen, die verstärkt oder vermindert gebildet werden und deren Veränderungen in Körperflüssigkeiten wie Blut, Plasma oder Serum nachgewiesen werden können.

Die Isolierung, Charakterisierung und Analyse intrazellulärer Bestandteile, vor allem von Nukleinsäuren (Ribonukleinsäuren, RNA und Desoxyribonukleinsäuren, DNA), ist von großer Bedeutung für die moderne Molekularbiologie. Spätestens seit der Erfindung der Polymerasekettenreaktion (PCR) im Jahre 1983 ist eine Vielzahl von Nukleinsäurediagnostik-Verfahren entwickelt worden, die beispielsweise zum Nachweis von Krankheiten und Krankheitserregern genutzt werden.

Im Stand der Technik wird solches Zellmaterial in den nachfolgenden Schritten, wie Probenaufbereitung, Extraktion, Konzentration, Isolierung, Reinigung, ggfs. reverse Transkription, Amplifikation und Detektion für die Diagnostik bereitgestellt.

Die Analyse genetischer Krebsveränderungen ist heutzutage von zentraler Bedeutung für das Patientenmanagement und die Therapieentscheidung. Die Bewertung des Mutationsprofils im Rahmen einer Krebserkrankung erfolgt meistens durch den Einsatz genetischer Tests. Dabei hat die Anwendung von Flüssigkeitsbiopsien aus Blut, die sich auf die Analyse der zirkulierenden Tumor-DNA (ctDNA), bzw. zellfreie DNA (cell free, cfDNA), oder RNA, z.B. solche in Exosomen oder zirkulierenden Tumorzellen (CTC, circulating tumour cells) bezieht, in den letzten Jahren in der klinischen Praxis enorm zugenommen, da sie eine kontinuierliche, minimal-invasive Überwachung des Tumorgenoms über den Krankheitsverlauf erlaubt. Mit der Verbreitung solcher Tests spezifisch für Flüssigkeitsbiopsien und ihrer Anwendung in der Routinediagnostik und wachsenden regulatorischen Anforderungen steigt gleichzeitig auch die Notwendigkeit, dass Assays validiert und nach gleichwertigen Standards durchgeführt werden.

Die Dokumentation der klinischen Validität eines Tests umfasst auch die Bewertung der analytischen Validität, welche durch die Parameter Genauigkeit, Sensitivität, Spezifität und Robustheit des Tests beschrieben werden. Einen großen Einfluss auf diese Parameter haben die prä-analytischen Variablen (bzw. Prozesse), welche Faktoren ohne direkte Krankheitsassoziation, aber sich auf die Integrität der Körperflüssigkeit oder des in dieser Körperflüssigkeit vorhandenen Biomarkers (Analyten) auswirken oder die Ergebnisse während der Analyse beeinflussen und technischen, biologischen oder umweltbedingten Ursprungs sein können. Im Bereich der Flüssigkeitsbiopsietests sind das z.B. die verwendete Extraktions- und Quantifizierungsmethode, Lagerungsbedingungen, PCR-Inhibition und Fragmentgrößenbias. Diese prä-analytischen Variablen können folglich zu Abweichungen zwischen den Laboren führen.

Aufgrund der leistungsfähigen Amplifikation von Tumor-DNA mittels Sequenzierverfahren von Nukleinsäuren, insbesondere PCR, "qPCR" (real-time quantitative Polymerase Chain Reaction), "digital droplet PCR" (ddPCR), oder "next generation sequencing" (NGS, bzw. Parallelsequenzierung wie "Massive Parallel Sequencing") ist in den letzten Jahren die so genannte nicht-invasive Liquid Biopsy in der Tumordiagnostik etabliert worden, wobei zum Beispiel zirkulierende freie DNA in einer Probe untersucht wird. Solche zirkulierende freie DNA (kurz: cfDNA) kann beispielsweise aus einer Krebs-/Tumorzelle stammen (so genannte: ctDNA).

Bei Krebspatienten liegt die durchschnittliche cfDNA Menge höher als in gesunden Probanden, insbesondere ist das cfDNA Plasma Level bei Krebspatienten im fortgeschrittenen Stadium höher als im milden/frühen Stadium. ctDNA macht z.B. 1% der cfDNA aus (0,01-90% der normalen cfDNA, je nach Stadium und Lokalisation des Tumors).

Es besteht jedoch ein hohes Bedürfnis an einem Standard, so dass nicht nur relative, sondern auch absolute diagnostische Aussagen getroffen werden können. Der Einsatz eines solchen Standards wird zum Beispiel in WO2018094183A1 oder PCT/EP2021/050962 der Anmelderin beschrieben.

Derzeit stehen z.B. für Mutationen, die es für die Tumordiagnostik nachzuweisen gilt, sowohl kommerzielle Tests als auch Laboreigene Tests (lab-developed test, LDT) zur Verfügung. Als Qualitätskontrollen für die Validierung bzw. Kalibrierung werden Labor-interne Standards wie z.B. Plasmid-DNA oder synthetische Oligonukleotide u.v.a. eingesetzt.

Von besonderem Nachteil ist, dass diese Labor-internen Standards die tatsächlichen Eigenschaften der aus Menschen oder Tieren erhaltenen Proben samt enthaltenen Biomarker oder Analyten oft ungenügend abbilden. Eine solche Probe betrifft das Untersuchungsmaterial wie es an einem Labor erhalten wird, also z.B. Blut, Gewebe, Rückenmarksflüssigkeit, Schleimhautabstriche, Sputum, Urin, Samen, Speichel, oder sonstige Probenarten.

Nachteilig ist ebenfalls, dass diese Labor-internen Standards zwecks Validierung oder Kalibrierung eines Gerätes oder eines Verfahrens in der in-vitro Diagnostik selbst verdünnt werden müssen und hierfür kein automatisierter Prozess zur Verfügung steht, sodass eine Standardisierung zumeist fehlerbehaftet ist. Ferner ist die Stabilität solcher Verdünnungen nicht gewährleistet und je nach Quelle wie z.B. Plasmid-DNA aus E. coli besteht die Gefahr einer DNA/RNA-Kontamination durch diese nicht humane oder nicht tierische Spezies, wobei bereits minimale Einträge kritisch sind. Folglich kann zum Beispiel die mittels PCR oder NGS ermittelte Kopienzahl mangels hinreichender Standardisierung relativ und absolut falsch sein.

Es ist offensichtlich, dass zur Bestimmung, Bewertung und Nachverfolgung der Analyseleistung, sowie zur Qualitätsüberwachung von Laboren interne und externe Kontrollen in Form eines Referenzmaterials erforderlich sind. Dabei sollen die Referenzmaterialien vergleichbare physikalische und biologische Eigenschaften zum eingesetzten Proben- bzw. Ausgangsmaterials aufweisen. Das wiederum bedeutet, dass nicht nur ein Fokus auf die Eigenschaft des Biomarkers gelegt wird, sondern eben auf die Probenmatrix, in der sich zum Beispiel ein nachzuweisender Biomarker/Analyt befinden kann. Das Zielmolekül der Flüssigkeitsbiopsietests, z.B. ct/cfDNA (circular tumor /cell free) befindet sich als zellfreie DNA im Blut und wird nach Blutabnahme, anschließender Plasma-Generierung aus dem abgenommenen Blut und mittels DNA-Extraktion isoliert. Jedoch nutzen bisherige Referenz- oder Kontrollmaterialien DNA-freies Plasma oder eine DNA-freie Plasma-artige Lösung als Basis, ggfs. weiter mit einem "Biomarker/Analyt of Interest" ergänzt, da Blut als solches zu viele Unbestimmtheiten aufweist. Diese Unbestimmtheiten können erst nach der Extraktion der Unbestimmtheiten analysiert werden. Dies wiederum macht das Blut als Kontrollmaterial unbrauchbar. Solche Unbestimmtheiten können sich aus der Probenbehandlung ergeben (z.B. Einwirkung von Hitze, Verunreinigung, Vollständigkeit, etc.). Als Konsequenz können jedoch die Prozesse der Probenbehandlung (Lagerung) und Plasma-Generierung oder weiterer Aufbereitungsmethoden nachteilig nicht validiert und bewertet werden. Es besteht daher das Bedürfnis eine Blutprobe als Referenzmaterial bereitzustellen, die weitgehend einer originären Blutprobe entspricht oder hinreichend simuliert, so dass ein solches Referenzmaterial mit einer Patienten-Blut(-plasma, -serum)probe verglichen werden kann. Die Patientenblutprobe kann hierbei beliebig behandelt worden sein.

Daher gilt es und es besteht die Aufgabe, die Standardisierung in der in-vitro Diagnostik, insbesondere solche mittels Sequenzierverfahren von Nukleinsäuren zu verbessern.

Überraschenderweise konnten die Erfinder feststellen, dass zu einem solchen Standard die Verwendung einer Blutprobe als Referenzmaterial in einer Diagnostik, oder in-vitro Diagnostik, welche ggfs. einen Stabilisator aufweist, die diagnostische Qualität erheblich verbessert, indem durch ein erfindungsgemäßes Referenzmaterial die
- Validität (Eine Messung ist valide, wenn sie tatsächlich das misst, was sie messen soll und somit glaubwürdige Ergebnisse liefert),
- Reliabilität (Die Reliabilität bezieht sich darauf, ob ein Testsystem bei wiederholter Durchführung zuverlässige Ergebnisse liefert), und
- Objektivität (Ein Testsystem ist objektiv, wenn keine ungewollten Einflüsse durch involvierte Personen entstehen) eines gegebenen Testsystems geprüft werden kann. Besonders vorteilhaft können auf diese Weise die Leistungsparameter der Messung überprüft werden. Insbesondere dann, falls eine humane oder tierische, insbesondere von Säugetieren, insbesondere, Rind, Schwein, oder Schaf, Vergleichsblutprobe vorliegt.

Die Aufgabe wird durch die vermittelte technische Lehre mindestens eines Patentanspruches gelöst.

Die Aufgabe der vorliegenden Erfindung ist daher die Bereitstellung eines verbesserten Verfahrens zur Durchführung einer Diagnostik, bzw. in-vitro Diagnostik, wobei zur Lösung dieser Aufgabe Blut oder in einer der nachstehenden Ausführungsformen als Referenzmaterial eingesetzt wird.

Daher betrifft die Erfindung die Verwendung von i.) Blut oder ii.) Blutzellen und Blutplasma oder Blutserum als Referenzmaterial in einer Probe in einer Diagnostik, bzw. in-vitro Diagnostik umfassend ein Sequenzierverfahren von Nukleinsäuren, wobei ggfs. ein Stabilisator hinzugefügt wird. In einer bevorzugten Ausführungsform der Erfindung ist der Stabilisator in der Weise zu wählen, dass kein Einfluss auf den zu bestimmenden Biomarker oder Analyten besteht.

Weiterhin betrifft die Erfindung die Verwendung eines Referenzmaterials aufweisend Blut in einer Probe in einer in-vitro Diagnostik umfassend ein Sequenzierverfahren von Nukleinsäuren, wobei Blutplasma oder Blutserum zu Blutzellen hinzugegeben wird.

Zudem betrifft die Erfindung ein Verfahren zur Herstellung von Blut als Referenzmaterial in einer Probe in einer in-vitro Diagnostik umfassend ein Sequenzierverfahren von Nukleinsäuren, wobei Blutplasma oder Blutserum zu Blutzellen hinzugegeben wird, und vorzugsweise ein oder mehrere Stabilisatoren hinzugefügt werden.

Solche erfindungsgemäß geeigneten Stabilisatoren sind vorzugsweise ausgewählt aus den Gruppen, wie vernetzende Stabilisatoren, alkoholische Stabilisatoren und / oder Antikoagulanzien. Bevorzugt sind insbesondere Formaldehyd, Glutaraldehyd, Glyoxal und Acrolein, oder Mischungen hieraus.

Weiterhin können solche Stabilisatoren verwendet werden, wie z.B. vernetzende Stabilisatoren, welche kovalente chemische Bindungen zwischen Proteinen herstellen, wie, nicht abschließend, ausgewählt aus der Gruppe Formaldehyd, Paraformaldehyd, Formalin, Glutaraldehyd, Osmiumtetraoxid, Imidoester Crosslinker, wie Dimethyl suberimidate (DMS), Acrolein, Glyoxal, Carbodiimide, oder alkoholische Stabilisatoren, wie, nicht abschließend, ausgewählt aus der Gruppe Ethanol, Methanol, Aceton, Essigsäure, 2-Phenoxyethanol, Diethylether oder Antikoagulazien / Chelatbildner, wie, nicht abschließend, ausgewählt aus der Gruppe EDTA (Ethylendiamintetraessigsäure), Heparine und Heparinoide, Citrat, Dimercaptobernsteinsäure, Dimercaptopropansulfonsäure, Acid-Citrate-Dextrose, Acetylaceton, Ethylendiamin, 2-(2-Aminoethylamino)ethanol, Diethylentriamin, Iminodiacetat, Triethylentetramin, Triaminotriethylamin, Nitrilotriacetat, Bis(salicyliden)ethylendiamin, Ethylendiaminotriacetat, Diethylentriaminpentaacetat, Triethylentetraminhexaacetat, 1,4,7,10-Tetraazacyclododecan-1,4,7,10-tetraacetat, Oxalat, Tartrat, Citrat, Dimethylglyoxim, 8-Hydroxychinolin, 2,2'-Bipyridin, 1,10-Phenanthrolin, 1,2-Bis(diphenylphosphino)ethan, Hirudine und Hirudinanaloga, Apixaban, Edoxaban, Rivaroxaban, Cumarine, Phenprocoumon, Warfarin, Dicumarol, Pentasaccharide, Plättchenaggregationshemmer, Acetylsalicylsäure (ASS), Clopidogrel, Ticagrelor, Prasugrel, GP-IIb-/IIIa-Antagonisten, Tirofiban, Eptifibatid, Bivalirudin, Rivaroxaban, Dabigatran etexilate, Apixaban, PPSB (Prothrombinkomplex), Antithrombin (AT III), Protein C, Desmopressin, DDAVP, Protaminchlorid, Vitamin K, Argatroban, Danaparoid, Danaparin, Lomoparan, oder ausgewählt aus der Gruppe Oxidierende Agenzien, Quecksilber basierte Stabilisatoren, B-5 (Quecksilberchlorid und Natriumacetat in wässriger Lösung), Zenker's Fixativ (Quecksilberchlorid, Kaliumdichromat, Natriumsulfat, Essigsäure, Wasser), Pikrinsäure basierte Stabilisatoren, wie Bouin-Lösung: Pikrinsäure, Essigsäure, Formaldehyd in wässriger Lösung, Ammoniumsulfat in wässriger Lösung, CARNOY Fixierlösung (Chloroform, ggfs. mit Eisen(III)Chlorid oder Formaldehyd-Alkohol-Essigsäure), Chromsäure, Chromessigsäure, Zinksalz Lösung.

In einer weiteren bevorzugten Ausführungsform betrifft die Erfindung solches Blutplasma oder Blutserum, welches nicht aus Menschen oder Tieren bereitgestellt wird, sondern eine Zusammensetzung aus Blutbestandteilen weitgehend entspricht, wobei die einzelnen Blutbestandteile jeweils hinzugefügt werden.

Ein erfindungsgemäßes Referenzmaterial kann daher folgende Zusammensetzung für das Blutserum oder Blutplasma aufweisen, wie in Tabelle 1 dargestellt, wobei neben Serum- oder Plasmaproteinen, insbesondere Albumin, ein oder mehrere Blutbestandteile hinzugefügt werden, insbesondere bis zu 5 Bestandteilen oder 10 Bestandteilen oder 20 Bestandteilen oder 30 Bestandteilen aus Tabelle 1. Insbesondere können neben Albumin bis zu 76 Bestandteile aus Tabelle 1 hinzugefügt werden. Die Konzentrationen der ausgewählten Bestandteile können jeweils variieren, oder aus Tabelle 1 jeweils ausgewählt werden.

**Tabelle 1: Referenzwerte für relevante Blutbestandteile in Plasma/Serum nach Hahn, Checkliste Innere Medizin, 2006**

| | **Stoffname / Blutbestandteil** | **Bevorzugte Konzentrationen** |
|---|---|---|
| 1. | ACTH | 2-11 pmol/l |
| 2. | Albumin | 35-55 g/l |
| 3. | Alpha-Amylase | < 100 U/l |
| 4. | Alphal-Fetoprotein (AFP) | < 10 ng/ml |
| 5. | Alkalische Phosphatase (AP) | 65-220 U/l |
| 6. | Ammoniak | m: 11-55 µmol/l |
| 7. | Antistreptolysintiter | < 200 IU/ml |
| 8. | Antithrombin (AT III) | 75-120 % |
| 9. | Bilirubin gesamt | 3,4-18,8 µmol/l |
| 10. | Bilirubin direkt | 0,9-5,1 µmol/l |
| 11. | Bilirubin indirekt | < 13,7 umol/l |
| 12. | Calcium | 2,3-2,6 mmol/l |
| 13. | Carcino-embryonales Antigen (CEA) | < 3 mg/l |
| 14. | Chlorid | 98-112 mmol/l |
| 15. | Cholesteringesamt | 3,1-6,5 mmol/l |
| 16. | HDL | > 1,0 mmol/l |
| 17. | LDL | < 4,0 mmol/l |
| 18. | Cholinesterase (CHE) | 3000-8000 U/l |
| 19. | C3-Komplement | 0,55-1,2 g/l |
| 20. | C4-Komplement | 0,2-0,5 g/l |
| 21. | Coeruloplasmin | 0,95-3,7 mmol/l |
| 22. | C-Peptid | 1,1-3,6 mg/l |
| 23. | C-reaktives Protein (CRP) | < 5 mg/l |
| 24. | Creatinkinase (CK) | <174 U/l |
| 25. | Creatinkinase-Isoenzym MB (CK-MB) | < 6 % der CK |
| 26. | Digoxin | 0,8-2,0 mg/l |
| 27. | Digitoxin | 15-25 mg/l |
| 28. | Eisen | 11-27 umol/l |
| 29. | alphal-Globulin | 1-4 g/l |
| 30. | alpha2-Globulin | 5-9 g/l |
| 31. | beta-Globulin | 6-11 g/l |
| 32. | gamma-Globulin | 8-15 g/l |
| 33. | Ferritin | 30-200 µg/l |
| 34. | Fibrinogen | 5,9-11,8 µmol/l |
| 35. | Folsäure | 3-15 ng/ml |
| 36. | Gesamteiweiß | 60-84g/l |
| 37. | Glukose nüchtern | 3,9-5,6mmol/l |
| 38. | GGT | 4-28 U/l |
| 39. | GOT | < 18 U/l |
| 40. | GPT | < 22 U/l |
| 41. | Haptoglobin | 0,2-2,04g/l |
| 42. | Harnsäure | 155-384 umol/l |
| 43. | Harnstoff | 1,7-9,3mmol/l |
| 44. | alpha-HBDH | 72-182 U/l |
| 45. | Immunglobulin G | 8-18g/l |
| 46. | Immunglobulin A | 0,9-4,5g/l |
| 47. | Immunglobulin M | 0,6-2,6g/l |
| 48. | Kalium | 3,5-5mmol/l |
| 49. | Kalzium | 2,3-2,6mmol/l |
| 50. | Kreatinin | 44-106 µmοl/l |
| 51. | Kupfer | 11-24 umol/l |
| 52. | Laktat | 0,63-2,44 mmol/l |
| 53. | LDH | 135-225 U/l |
| 54. | LAP | 16-32 U/l |
| 55. | Lipase | 30-180 U/l |
| 56. | Lipoprotein (a) | < 300 mg/l |
| 57. | Magnesium | 0,7-1,6 mmol/l |
| 58. | Natrium | 135-150 mmol/l |
| 59. | Phosphat | 0,77-1,55 mmol/l |
| 60. | Prostataspez. Antigen | < 3 µg/l |
| 61. | Rheumafaktor | < 20 IU/ml |
| 62. | Theophyllin | 10-20mg/l |
| 63. | TSH basal | 0,3-3,5 mU/l |
| 64. | freies Thyroxin (FT4) | 7-30 pmol/l |
| 65. | freies Trijodthyronin (FT3) | 4,6-9,2 pmol/l |
| 66. | TBG | 12-30 gg/ml |
| 67. | Thyreoglobulin | < 50 ng/ml |
| 68. | Transferrin | 2,0-4,0g/l |
| 69. | Triglyzeride | 0,83-2,3mmol/l |
| 70. | Vitamin A | 0,7-2,8 µmol/l |
| 71. | Vitamin B12 | 229-812 pmol/l |
| 72. | Vitamin D | 700-3100 U/l |
| 73. | Vitamin E | 12-48 umol/l |
| 74. | Fragmentierte DNA, cfDNA | 1-17 ng DNA/ml |
| 75. | Genomische DNA, gDNA | < 2µg/ml |
| 76. | Extrazelluläre Vesikel | 2 * 10¹⁰/mL |
| 77. | RNA | 1-1000 ng/ml |

Alle in einer wässrigen Lösung. Alle genannten Proteine werden auch Serum- oder Plasmaprotein genannt.

In einer bevorzugten Ausführungsform ist die Zusammensetzung frei von Antikörpern, insbesondere frei von Immunglobulinen und / oder frei von DNA, RNA oder extrazellulären Vesikeln.

Eine solche bereitgestellte Blutprobe wird nachstehend erfindungsgemäßes Referenzmaterial genannt.

In einer weiteren bevorzugten Ausführungsform weist das hinzugegebene Blutplasma oder Blutserum definierte Werte an mindestens einem Inhaltsstoff auf, ausgewählt aus der Gruppe gDNA, fragmentierte DNA, tumorassoziiert und nicht tumorassoziiert, methylierte oder nicht methylierte cfDNA, histongebundene DNA, zirkuläre DNA, mitochondriale DNA, einzelsträngige oder doppelsträngige DNA, ctDNA, RNA, methylierte oder nicht methylierte RNA, miRMA, tRNA, scRNA, rRNA, mRNA, Tumornukleinsäure, gespikte Nukleinsäure, Exosom, Proteine, Peptide, extrazelluläre Vesikel, Exosomen, insbesondere mit den folgenden Bereichen 1-1500 ng DNA/ml, insbesondere mit einem Anteil an cfDNA bei Schwangeren von 2-15 % an fetaler cfDNA, 1-1000 ng RNA/ml.

Weiterhin weist das hinzugegebene Blutplasma oder Blutserum definierte Werte an mindestens einem biologischen Material auf, ausgewählt aus der Gruppe Zellen, eukaryotische Zellen Viren, Pilze, Pilzsporen, Prionen, Bakterien, Parasiten, Tumorzellen, zirkulierende Tumorzellen (CTC), zirkulierende Endothelzellen (CTEC), insbesondere mit den folgenden Bereichen 1-100.000 Viren/ml, 1-100.000 Bakterien/ml, 1-100.000 Pilzen/ml, 1-100.000 Pilzsporen/ml, 1-100.000 Parasiten/ml, 1-1.000 Tumorzellen/ml oder 1-1.000 Endothelzellen/ml.

Die vorgenannten Ausführungsformen des erfindungsgemäßen Referenzmaterials erlauben vorteilhaft die Simulation von Blutproben von beispielsweise kranken Patienten und anderen Zuständen.

Weiterhin ist bevorzugt, dass fragmentierte (zellfreie) DNA (cfDNA), die in verschiedenen Größen vorkommen kann, bevorzugt in den Größenbereichen 140-180 Basenpaare (bp), 310-350 bp, 480-520 bp verwendet wird. Die Erfinder konnten feststellen, dass gerade für Sequenzierverfahren von Nukleinsäuren die Größe als auch Größenverteilung solcher cfDNA in einem Referenzmaterial kritisch ist.

In einer weiteren Ausführungsform der Erfindung können große cfDNA Fragmente vorzugsweise in einer Größenordnung von 2.000 bis 15.000 bp verwendet werden, während mitochondriale DNA zwischen 40-300bp verwendet wird. Extrachromosomale zirkuläre DNA weist eine Größe zwischen 30-20.000 bp auf.

Weiterhin ist bevorzugt, dass RNA, insbesondere miRNA, die in verschiedenen Größen vorkommen kann, bevorzugt in den Größenbereichen 10-250 bp, insbesondere 10-500 bp verwendet wird. Die Erfinder konnten feststellen, dass gerade für Sequenzierverfahren von Nukleinsäuren die Größe als auch Größenverteilung solcher RNA, insbesondere miRNA in einem Referenzmaterial kritisch ist.

"Blut" im Sinne dieser Erfindung kann Vollblut sein, welche Blutzellen und Blutserum aufweisen. Insbesondere kann das Blut einem Menschen oder einem Tier entnommen werden.

"Blutzellen" sind im Sinne dieser Erfindung solche, die nach Abtrennen des Blutserums zum Beispiel nach Zentrifugation im Rückstand verbleiben und Erythrozyten, Leukozyten, Thrombozyten u.a. umfassen.

In einer bevorzugten Ausführungsform der Erfindung beträgt der Anteil an Blutzellen 15 - 70 Gew. %, vorzugsweise 37 - 54 Gew. % an dem erfindungsgemäßen Referenzmaterial.

Weiterhin ist bevorzugt an dem erfindungsgemäßen Referenzmaterial, dass 99 Gew. % Erythrozyten (4,5 - 5,9 x 10^6 Zellen/ µl Blut sind, wobei der unterste Wert für Frauen und der oberste Wert für Männer gilt.

Weiterhin ist bevorzugt an dem erfindungsgemäßen Referenzmaterial, dass < 1 % Leukozyten (ca. 4.000 - 10.000 Zellen / µl Blut für Erwachsene und für Kinder: bis zu 17.000 Zellen / µl Blut, Säuglinge bis zu 30.000 Zellen / µl Blut) und < 1 % Thrombozyten (ca. 150.000 - 400.000 Zellen/ µl Blut) .

"Blutplasma" im Sinne dieser Erfindung ist durch Zentrifugieren von Blut und Abtrennen des Überstandes erhältlich, wobei das Blut zuvor mit einem Gerinnungshemmer, wie zum Beispiel Natriumcitrat oder EDTA versetzt wurde. Das Blutplasma enthält zumeist alle Gerinnungs- und Fibrinolysefaktoren in aktiver Form, sowie die cfDNA, die insbesondere bei der Anwendung der Flüssigkeitsbiopsietests weiter analysiert wird.

"Blutserum" im Sinne dieser Erfindung ist durch Zentrifugieren von Blut und Abtrennen des Überstandes erhältlich. Im Unterschied zum Blutplasma sind keine Gerinnungshemmer enthalten.

Im Sinne dieser Erfindung kann ebenfalls Blutserum oder Blutplasma verwendet werden, insbesondere solche welche im Wesentlichen folgende Bestandteile aufweisen:

**Tabelle 2: Beispiel einer Zusammensetzung und Konzentrationen der Inhaltsstoffe eines erfindungsgemäßen Serums oder Plasmas (Lösungsmittel ist Wasser)**

| **Stoffname / Blutbestandteil** | **Konzentration** |
|---|---|
| Plasmaproteine, insb. Albumin | 50,3 g/l |
| Elektrolyte (Na⁺) | 89,6 mmol/l |
| Elektrolyte (K⁺) | 2,0 mmol/l |
| Elektrolyte (Ca⁺) | 0,025 mmol/l |
| Elektrolyte (Cl⁻) | 105,8 mmol/l |
| Elektrolyte (PO₄³⁻) | 6,7 mmol/l |

**Tabelle 3: Beispiel einer Zusammensetzung und Konzentrationen der Inhaltsstoffe eines erfindungsgemäßen Serums oder Plasmas mit Zugabe von Stabilisatoren (Lösungsmittel ist Wasser)**

| **Stoffname** | **Konzentration** |
|---|---|
| Plasmaproteine, insb. Albumin | 50,3 g/l |
| Elektrolyte (Na⁺) | 89,6 mmol/l |
| Elektrolyte (K⁺) | 2,0 mmol/l |
| Elektrolyte (Ca⁺) | 0,025 mmol/l |
| Elektrolyte (Cl⁻) | 105,8 mmol/l |
| Elektrolyte (PO₄³⁻) | 6,7 mmol/l |
| Organische Säuren (Citrat) | 0,008 mmol/l |
| EDTA | 9,7183 mmol/l |

**Tabelle 4: Beispiel einer Zusammensetzung und Konzentrationen der Inhaltsstoffe eines erfindungsgemäßen Serums oder Plasmas mit Zugabe von Stabilisatoren und zugegebenem Inhaltsstoff, welcher einem Analyten entspricht (Lösungsmittel ist Wasser)**

| **Stoffname** | **Konzentration** |
|---|---|
| Plasmaproteine (z.B. Albumin) | 50,3 g/l |
| Elektrolyte (Na⁺) | 89,6 mmol/l |
| Elektrolyte (K⁺) | 2,0 mmol/l |
| Elektrolyte (Ca⁺) | 0,025 mmol/l |
| Elektrolyte (Cl⁻) | 105,8 mmol/l |
| Elektrolyte (PO₄³⁻) | 6,7 mmol/l |
| Organische Säuren (Citrat) | 0,008 mmol/l |
| EDTA | 9,7183 mmol/l |
| Fragmentierte DNA | 80 ng DNA/ml |
| Extrazelluläre Vesikel | 2 * 10¹⁰/mL |
| RNA | 1000 ng/ml |
| Viren | 1000/ml |
| Pilzsporen | 1000/ml |
| Bakterien | 1000/ml |
| Genomische DNA | |

In einer weiteren Ausführungsform kann daher zu dem erfindungsgemäßen Referenzmaterial eine oder mehrere chemische Substanzen hinzugefügt werden. Solche chemischen Substanzen können nicht abschließend sein, insbesondere mindestens ein organisches Molekül, welches neben Kohlenstoff (C) und Wasserstoff (H) Heteroatome enthalten kann, wie Sauerstoff (O), Stickstoff (N), Schwefel (S) oder Phosphor (P). Die chemischen Substanzen können lineare und/oder ringförmige Kohlenstoffketten samt Heteroatomen aufweisen. Bevorzugt sind organische Moleküle mit weniger als 1.000 g/mol. Eine solche chemische Substanz kann ein Biomarker, Analyt, Wirkstoff, Antikörper, Antigen oder Medikament sein. Insbesondere kann ein Inhaltsstoff hinzugefügt werden, ausgewählt aus der Gruppe gDNA, fragmentierte DNA, tumorassoziiert und nicht tumorassoziiert, methylierte oder nicht methylierte cfDNA, histongebundene DNA, zirkuläre DNA, mitochondriale DNA, einzelsträngige oder doppelsträngige DNA, ctDNA, RNA, methylierte oder nicht methylierte RNA, miRMA, tRNA, scRNA, rRNA, mRNA, Tumornukleinsäure, gespikte Nukleinsäure, Exosom, Proteine, Peptide, extrazelluläre Vesikel, Exosomen.

In einer weiteren Ausführungsform kann zu dem erfindungsgemäßen Referenzmaterial ein oder mehrere biologische Materialien hinzugefügt werden, solche wie, ggfs. lebens- oder funktionsfähig, wie Zellen, eukaryotische Zellen Viren, Pilze, Pilzsporen, Prionen, Bakterien, Parasiten, Tumorzellen, zirkulierende Tumorzellen (CTC), zirkulierende Endothelzellen (CTEC).

Im Sinne dieser Erfindung bedeutet "Tumornukleinsäure" eine beliebige und bekannte humane Wildtypsequenz. Solche Tumornukleinsäuren können Datenbanken, wie
COSMIC: https://cancer.sanger.ac.uk/cosmic,
Targeted Cancer Care: http://targetedcancercare.massgeneral.org/My-Trial-Guide/Diseases/Lung-Cancer/KRAS/G12C-(c-34G-T).aspx, oder
OncoKB: https://oncokb.org/, My Cancer Genome:
   https://www.mycancergenome.org/, National Center for Biotechnology Information (NCBI)
   https://www.ncbi.nlm.nih.gov/gene/ entnommen werden.

In einer bevorzugten Ausführungsform beträgt der Gehalt an einer Tumornukleinsäure, vorzugsweise 5 - 1000 ng, insbesondere 400 ng DNA in Serum oder Plasma, wie z.B. 400 ng/5ml, entspricht 80 ng/ml, entspricht 0,08 ng/µl DNA in Serum oder Plasma.

Weiterhin ist bevorzugt, dass die Tumornukleinsäure eine Größe von 30 bp bis 500 bp aufweist.

Im Sinne dieser Erfindung bedeutet "gespikte Nukleinsäure" eine solche Sequenz, die gegenüber einer humanen Wildtypsequenz einer Nukleinsäure ein oder mehrere Mutationen aufweist. Hierbei können gegenüber der humanen Wildtypsequenz bekannte Mutationssequenzen eingesetzt oder künstliche Mutationen eingeführt werden. Weiterhin ist bevorzugt, dass die gespikte Nukleinsäure mindestens einen Tumormarker, z.B. Onkogen, aufweist mit bekannten Mutationen, insbesondere ein Onkogen ausgewählt aus der Gruppe ABI1,ABL1,ABL2,ACKR3,ACSL3,ACVR1,ACVR2A,AFDN,AFF1,AFF3,AFF4,AK T1,AKT2,ALK,AMER1,APC,APOBEC3B,AR,ARHGAP26,ARHGEF12,ARID1A,ARI D1B,ARID2,ARNT,ASPSCR1,ASXL1,ATF1,ATIC,ATM,ATP1A1,ATP2B3,ATR,A TRX,AXIN1,AXIN2,B2M,BAP1,BARD1,BAX,BCL10,BCL11A,BCL11B,BCL2,BC L3,BCL6,BCL7A,BCL9,BCL9L,BCOR,BCORL1,BCR,BIRC3,BLM,BMPR1A,BRAF ,BRCA1,BRCA2,BRD3,BRD4,BRIP1,BTG1,BTK,BUB1B,CACNA1D,CALR,CAMTA 1,CANT1,CARD11,CARS,CASP8,CBFA2T3,CBFB,CBL,CBLB,CBLC,CCDC6,CCN B1IP1,CCND1,CCND2,CCND3,CCNE1,CD274,CD74,CD79A,CD79B,CDC73,CDH 1,CDH11,CDK12,CDK4,CDK6,CDKN1B,CDKN2A,CDKN2C,CDX2,CEBPA,CHCHD7 ,CHD4,CHEK2,CIC,CIITA,CLIP1,CLTC,CLTCL1,CNBP,CNOT3,CNTRL,COL1A 1,COL2A1,CREB1,CREB3L1,CREB3L2,CREBBP,CRLF2,CRTC1,CRTC3,CSF3R, CTCF,CTNNB1,CUX1,CXCR4,CYLD,DAXX,DCTN1,DDB2,DDIT3,DDR2,DDX10,D DX3X,DDX5,DDX6,DEK,DICER1,DNAJB1,DNM2,DNMT3A,DROSHA,EBF1,EGFR, EIF3E,EIF4A2,ELF4,ELK4,ELL,EML4,EP300,EPAS1,EPS15,ERBB2,ERBB3, ERBB4,ERC1,ERCC2,ERCC3,ERCC4,ERCC5,ERG,ESR1,ETNK1,ETV1,ETV4,ET V5,ETV6,EWSR1,EXT1,EXT2,EZH2,EZR,FANCA,FANCC,FANCD2,FANCE,FANC F,FANCG,FAS,FAT1,FAT4,FBXO11,FBXW7,FCGR2B,FCRL4,FES,FEV,FGFR1, FGFR1OP,FGFR2,FGFR3,FGFR4,FH,FHIT,FIP1L1,FLCN,FLI1,FLT3,FLT4,F OXA1,FOXL2,FOXO1,FOXO3,FOXO4,FOXP1,FSTL3,FUBP1,FUS,GAS7,GATA1, GATA2,GATA3,GNA11,GNAQ,GNAS,GOLGA5,GOPC,GPC3,GPHN,GRIN2A,H3F3A ,H3F3B,HERPUD1,HEY1,HIF1A,HIP1,HIST1H3B,HIST1H4I,HLA-A,HLF,HMGA1,HMGA2,HNF1A,HNRNPA2B1,HOOKS,HOXA11,HOXA13,HOXA9,HO XC11,HOXC13,HOXD11,HOXD13,HRAS,HSP90AA1,HSP90AB1,IDH1,IDH2,IGH ,IGK,IGL,IKBKB,IKZF1,IL2,IL21R,IL6ST,IL7R,IRF4,IRS4,ITK,JAK1,J AK2,JAK3,JUN,KAT6A,KAT6B,KCNJ5,KDM5A,KDM5C,KDM6A,KDR,KDSR,KEAP 1,KIF5B,KIT,KLF4,KLF6,KLK2,KMT2A,KMT2C,KMT2D,KNL1,KRAS,KTN1,LA SP1,LATS1,LATS2,LCK,LEF1,LIFR,LMNA,LMO1,LMO2,LPP,LRIG3,LRP1B,L YL1,LZTR1,MAF,MAFB,MALT1,MAML2,MAP2K1,MAP2K2,MAP2K4,MAP3K1,MAP 3K13,MAPK1,MAX,MDM2,MDM4,MECOM,MED12,MEN1,MET,MITF,MLF1,MLH1,M LLT1,MLLT10,MLLT11,MLLT3,MLLT6,MN1,MPL,MRTFA,MSH2,MSH6,MS12,MS N,MTCP1,MTOR,MUC1,MUTYH,MYB,MYC,MYCL,MYCN,MYD88,MYH11,MYH9,MYO 5A,MYOD1,NAB2,NBN,NCOA1,NCOA2,NCOA4,NCOR1,NCOR2,NDRG1,NF1,NF2, NFATC2,NFE2L2,NFIB,NFKB2,NFKBIE,NIN,NKX2-1, NONO,NOTCH1,NOTCH2,NPM1,NR4A3,NRAS,NRG1,NSD1,NSD2,NSD3,NT5C2,N TRK1,NTRK3,NUMA1,NUP214,NUP98,NUTM1,NUTM2B,NUTM2D,OLIG2,P2RY8, PAFAH1B2,PALB2,PATZ1,PAX3,PAX5,PAX7,PAX8,PBRM1,PBX1,PCM1,PDCD1 LG2,PDE4DIP,PDGFB,PDGFRA,PDGFRB,PER1,PHF6,PHOX2B,PICALM,PIK3CA ,PIK3CB,PIK3R1,PIM1,PLAG1,PLCG1,PML,PMS2,POLD1,POLE,POLQ,POT1, POU2AF1,POU5F1,PPARG,PPFIBP1,PPM1D,PPP2R1A,PPP6C,PRCC,PRDM1,PR DM16,PREX2,PRF1,PRKACA,PRKAR1A,PRRX1,PSIP1,PTCH1,PTEN,PTK6,PTP N11,PTPN13,PTPRB,PTPRC,PTPRK,PTPRT,QKI,RABEP1,RAC1,RAD21,RAD51 B,RAF1,RANBP2,RAP1GDS1,RARA,RB1,RBM10,RBM15,RECQL4,REL,RET,RHO A,RHOH,RMI2,RNF213,RNF43,ROS1,RPL10,RPL22,RPL5,RPN1,RSPO2,RSPO 3,RUNX1,RUNX1T1,SALL4,SBDS,SDC4,SDHA,SDHAF2,SDHB,SDHC,SDHD,SET ,SETBP1,SETD2,SF3B1,SFPQ,SFRP4,SH2B3,SH3GL1,SIX1,SLC34A2,SLC45 A3,SMAD2,SMAD3,SMAD4,SMARCA4,SMARCB1,SMARCD1,SMARCE1,SMO,SND1, SOCS1,SOX2,SPEN,SPOP,SRC,SRSF2,SRSF3,SS18,SS18L1,SSX1,SSX2,SSX 4,STAG2,STAT3,STAT5B,STAT6,STIL,STK11,STRN,SUFU,SUZ12,SYK,TAF1 5,TAL1,TAL2,TBL1XR1,TBX3,TCEA1,TCF12,TCF3,TCF7L2,TCL1A,TENT5C, TERT,TET1,TET2,TFE3,TFEB,TFG,TGFBR2,TLX1,TLX3,TMEM127,TMPRSS2, TNFAIP3,TNFRSF14,TNFRSF17,TOP1,TP53,TP63,TPM3,TPM4,TPR,TRA,TRA F7,TRB,TRD,TRIM24,TRIM27,TRIM33,TRIP11,TRRAP,TSC1,TSC2,TSHR,U2 AF1,UBR5,USP6,USP8,VHL,WAS,WDCP,WIF1,WRN,WT1,WWTR1,XPA,XPC,XPO 1,YWHAE,ZBTB16,ZFHX3,ZMYM2,ZNF331,ZNF384,ZNF521,ZRSR2,A1CF,ACS L6, AKAP9, AKT3, ALDH2, ANK1, ARAF, ARHGAP5, ARHGEF10, ARHGEF10L, ASXL2 ,BAZ1A, BCL2L12, BCLAF1, BIRC6, BMP5, C15orf65, CASP3, CASP9, CCNC, CCR 4, CCR7, CD209, CD28, CDH10, CDH17, CDKN1A, CEP89, CHD2, CHIC2, CHST11, C LP1,CNBD1,CNTNAP2,COL3A1,COX6C,CPEB3,CRNKL1,CSF1R,CSMD3,CTNNA2 , CTNND1, CTNND2, CUL3, CYP2C8, CYSLTR2, DCAF12L2, DCC, DGCR8, DUX4L1, E CT2L,EED,EIF1AX,ELF3,ELN,EPHA3,EPHA7,FAM131B,FAM135B,FAM47C,FA T3,FBLN2,FEN1,FKBP9,FLNA,FNBP1,FOXR1,GLI1,GMPS,GPC5,GRM3,HMGN2 P46, ID3, IGF2BP2, ISX, ITGAV, JAZF1, KAT7, KIAA1549, KNSTRN, LARP4B, LC P1, LEPROTL1, LHFPL6, LSM14A, MACC1, MALAT1, MB21D2, MDS2, MGMT, MNX1, M UC16,MUC4,N4BP2,NACA,NBEA,NCKIPSD,NTHL1,OMD,PABPC1,PCBP1,PMS1, POLG,PRDM2,PRKCB,PRPF40B,PTPN6,PTPRD,PWWP2A,RAD17,RALGDS,RFWD3 ,RGPD3, RGS7, R0B02, S100A7, SEPT5, SEPT6, SEPT9, SETD1B, SETDB1, SGK1, SHTN1,SIRPA,SIX2,SKI,SMC1A,SNX29,SOX21,SPECC1,SRGAP3,STAG1,TEC ,TFPT, TFRC, THRAP3, TNC, USP44, VAV1, VTI1A, WNK2, ZCCHC8, ZEB1, ZMYM3, ZNF429,ZNF479,ZNRF3.

Weiterhin können solche Tumormarkersequenzen oder Onkogene weitere künstliche Mutationen aufweisen.

Im Sinne dieser Erfindung bedeutet "Validierung", dass anhand des erfindungsgemäßen Referenzmaterials als Referenzprobe die bestimmungsgemäße Diagnostik, bzw. in-vitro Diagnostik durchgeführt wird, insbesondere zur Durchführung einer Kalibrierung unter Berücksichtigung der Geräteparameter oder zur Erstellung einer Eichkurve.

In einer bevorzugten Ausführungsform der Erfindung ist die in-vitro Diagnostik eine Liquid Biopsy, wobei vorzugsweise mittels PCR aus einer Blutprobe gegen die erfindungsgemäße Blutprobe gemessen wird oder die erfindungsgemäße Blutprobe zur Kalibrierung, Validierung, der Diagnostik, oder in-vitro Diagnostik herangezogen wird.

Überraschender Weise können auf diese Weise vorteilhaft bereits geringe Mengen an Probennukleinsäure z.B. aus einer Blutprobe mit hinreichender Spezifität und Sensitivität zu dem erfindungsgemäßen Referenzmaterial erfasst werden. Dies erlaubt im Fall einer Liquid Biopsy besonders vorteilhaft eine frühzeitige Aussage über die Tumoraktivität, insbesondere die Wahrscheinlichkeit einer Metastasierung. Eine bevorzugte Probennukleinsäure ist cfDNA oder ctDNA eines Patienten oder Probanden.

Daher betrifft die Erfindung ebenfalls die vorteilhafte Verwendung des Referenzmaterials in einem Verfahren zur Bestimmung der Allelfrequenz und / oder Mutationsrate und/oder zur absoluten Quantifizierung mittels Kopienzahlbestimmung mindestens einer Probennukleinsäure mittels eines Sequenzierverfahrens für Nukleinsäuren. Besonders vorteilhaft kann anhand der Kalibrierung bzw. Validierung eine quantitative Bestimmung der Probennukleinsäure erfolgen.

Weiterhin betrifft die Erfindung die vorteilhafte Verwendung des Referenzmaterials in einem Verfahren zur präanalytischen Evaluierung (Transport, Verarbeitung einer Blutprobe, Lagerung/Wiederholte Verwendung z.B. Gefrier-/Auftauzyklen, Extraktionsverfahren), analytischen Evaluierung (Genauigkeit / Präzision, Wiederholbarkeit / Reproduzierbarkeit, Analytische Spezifität / Sensitivität, Detektionslimit, Quantifizierungslimit, Leerwertgrenze, Linearität, Interferenz, Robustheit).

Das Bereitstellen des erfindungsgemäßen Referenzmaterials erlaubt besonders vorteilhaft, die Nachweisgrenze der Probennukleinsäuren zu validieren. Eine besonders vorteilhafte Anwendung betrifft daher die Validierung von Geräten zur Durchführung einer Polymerase-Chain-Reaction (PCR), insbesondere next generation sequencing (NGS).

Besonders vorteilhaft kann auf diese Weise eine exakt vorgegebene Menge an DNA-Material bzw. Konzentration in das erfindungsgemäße Referenzmaterial eingebracht werden, wobei dieses Referenzmaterial eine Patientenblutprobe simuliert und insbesondere für die Durchführung einer Tumordiagnostik, Schwangerschaftsdiagnostik, heriditäre genetische Diagnostik oder Infektionsdiagnostik hochgradig geeignet ist.

"In-vitro Diagnose" im Sinne dieser Erfindung bedeutet jedwede Diagnose außerhalb des menschlichen oder tierischen Körpers (ex vivo) unter Bereitstellung geeigneter Assays und Geräte. Bevorzugt ist die Aussage über eine Krankheit oder einen Zustand eines Patienten.

Im Rahmen dieser Erfindung wird unter "Patient" ein beliebiger Proband verstanden.

Nachfolgend wird die Erfindung durch Beispiele erläutert. Die Erfindung ist jedoch nicht auf die Beispiele beschränkt, sondern grundsätzlich universell anwendbar.

### Beispiel 1:

Die einzelnen Bestandteile aus Tabelle 1 können mit Blutzellen vermengt werden und auf diese Weise ein geeignetes Referenzmaterial bereitgestellt werden.

### Beispiel 2:

Zugesetzte DNA in dem Referenzmaterial kann unter anderem wie folgt fragmentiert hinzugegeben werden, um die biologische Situation eines Menschen so nah wie möglich abbilden zu können.

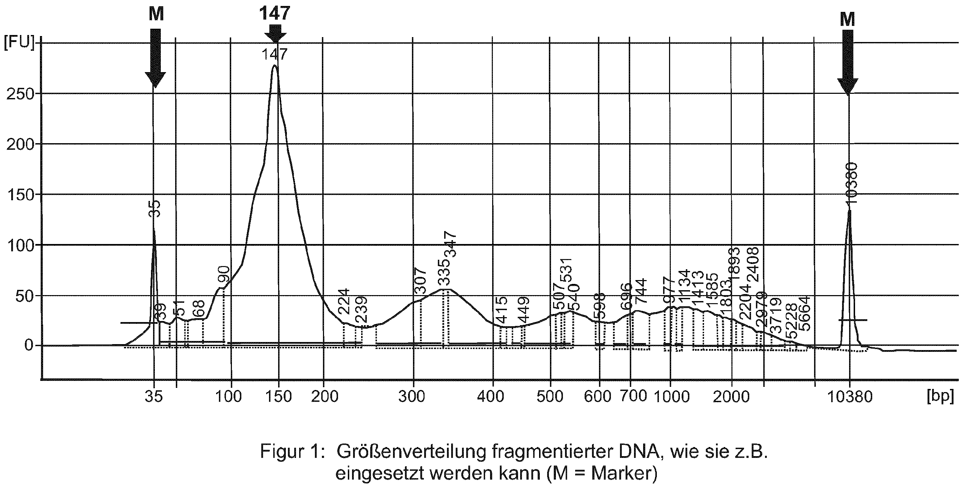

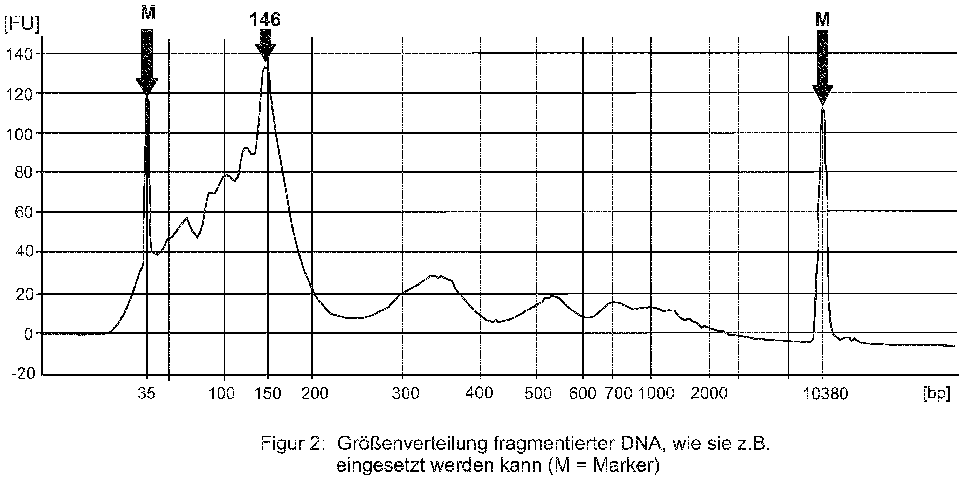

## Patentansprüche

1. Verwendung eines Referenzmaterials aufweisend Blut in einer Probe in einer in-vitro Diagnostik umfassend ein Sequenzierverfahren von Nukleinsäuren, wobei ein Stabilisator hinzugefügt wird.

2. Verwendung eines Referenzmaterials aufweisend Blutzellen und Blutplasma oder Blutserum in einer Probe in einer in-vitro Diagnostik umfassend ein Sequenzierverfahren von Nukleinsäuren, wobei ein Stabilisator hinzugefügt wird.

3. Verwendung eines Referenzmaterials aufweisend Blutzellen und Blutplasma oder Blutserum in einer Probe in einer in-vitro Diagnostik umfassend ein Sequenzierverfahren von Nukleinsäuren, wobei das Blutplasma oder Blutserum aus Albumin und 5 Bestandteilen oder bis zu 30 Bestandteilen der Tabelle 1 zusammengesetzt ist.

4. Verwendung eines Referenzmaterials aufweisend Blutzellen und Blutplasma oder Blutserum in einer Probe in einer in-vitro Diagnostik umfassend ein Sequenzierverfahren von Nukleinsäuren, wobei das Blutplasma oder Blutserum aus Albumin und bis zu 76 Bestandteilen der Tabelle 1 zusammengesetzt ist.

5. Verwendung eines Referenzmaterials aufweisend Blutzellen und Blutplasma oder Blutserum in einer Probe in einer in-vitro Diagnostik umfassend ein Sequenzierverfahren von Nukleinsäuren nach Anspruch 3 oder Anspruch 4, wobei das Blutplasma oder Blutserum nicht aus Menschen oder Tieren bereitgestellt wird.

6. Verfahren zur Herstellung eines Referenzmaterials in einer Probe in einer in-vitro Diagnostik umfassend ein Sequenzierverfahren von Nukleinsäuren nach einem der Ansprüche 1 bis 5, wobei Blutplasma oder Blutserum zu Blutzellen hinzugegeben wird, und ein Stabilisator hinzugefügt wird.

7. Verwendung oder Verfahren nach einem der vorherigen Ansprüche, wobei das Blutplasma oder Blutserum definierte Werte an Inhaltsstoffen und / oder biologischen Material aufweist.

8. Verwendung oder Verfahren nach einem der vorherigen Ansprüche, wobei das Blutplasma oder Blutserum mindestens einen Inhaltsstoff aufweist, ausgewählt aus der Gruppe gDNA, fragmentierte DNA tumorassoziiert und nicht tumorassoziiert, methylierte oder nicht methylierte cfDNA, histongebundene DNA, zirkuläre DNA, mitochondriale DNA, einzelsträngige oder doppelsträngige DNA, ctDNA, RNA, methylierte oder nicht methylierte RNA, miRMA, tRNA, scRNA, rRNA, mRNA, Tumornukleinsäure, gespikte Nukleinsäure, Exosom, Proteine, Peptide, extrazelluläre Vesikel, Exosomen, insbesondere mit den folgenden Bereichen 1-1500 ng DNA/ml, insbesondere mit einem Anteil an cfDNA bei Schwangeren von 2-15 % an fetaler cfDNA, 1-1000 ng RNA/ml.

9. Verwendung oder Verfahren nach einem der vorherigen Ansprüche, wobei das Blutplasma oder Blutserum mindestens ein biologisches Material aufweist, ausgewählt aus der Gruppe Zellen, eukaryotische Zellen Viren, Pilze, Pilzsporen, Prionen, Bakterien, Parasiten, Tumorzellen, zirkulierende Tumorzellen (CTC), zirkulierende Endothelzellen (CTEC), insbesondere mit den folgenden Bereichen 1-100.000 Viren/ml, 1-100.000 Bakterien/ml, 1-100.000 Pilzen/ml, 1-100.000 Pilzsporen/ml, 1-100.000 Parasiten/ml, 1-1.000 Tumorzellen/ml oder 1-1.000 Endothelzellen/ml.

10. Verwendung oder Verfahren nach einem der vorherigen Ansprüche, wobei der Stabilisator ausgewählt wird aus der Gruppe bestehend aus vernetzenden Stabilisatoren, alkoholische Stabilisatoren und / oder Antikoagulanzien, insbesondere Formaldehyd, Glutaraldehyd, Glyoxal und Acrolein.

11. Verwendung oder Verfahren nach einem der vorherigen Ansprüche, wobei mindestens eine weitere chemische Substanz und / oder mindestens ein biologisches Material hinzugefügt wird, insbesondere i.) Biomarker, Analyt, Wirkstoff, Antikörper, Antigen oder Medikament oder ii.) Zellen, eukaryotische Zellen Viren, Pilze, Pilzsporen, Prionen, Bakterien, Parasiten, Tumorzellen, zirkulierende Tumorzellen (CTC), zirkulierende Endothelzellen (CTEC).

12. Verwendung oder Verfahren nach einem der vorherigen Ansprüche, **dadurch gekennzeichnet, dass** mithilfe des Referenzmaterials eine Validierungs- oder Kalibrierungskurve erstellt wird.

13. Verwendung oder Verfahren nach einem der vorherigen Ansprüche, **dadurch gekennzeichnet, dass** das Sequenzierverfahren von Nukleinsäuren mittels PCR, qPCR (real-time quantitative Polymerase Chain Reaction), digital droplet PCR (ddPCR) oder "next generation sequencing" (NGS) durchgeführt wird.

14. Verwendung oder Verfahren nach einem der vorherigen Ansprüche, **dadurch gekennzeichnet, dass** das Referenzmaterial eine Patientenblutprobe simuliert, insbesondere zur Durchführung einer Tumordiagnostik, Schwangerschaftsdiagnostik, heriditäre genetische Diagnostik oder Infektionsdiagnostik.
